# EUROPEAN PATENT APPLICATION

(11) **EP 2 329 786 A2**
(43) Date of publication of application: **08.06.2011**
(21) Application number: 10183665.8
(22) Date of filing: 30.09.2010
(51) Int. Cl.: A61B 19/00

(54) **Guided surgery**

(30) Priority: 01.10.2009 US 247607 P; 11.05.2010 US 333519 P
(71) Applicant: Navotek Medical Ltd., 20692 Yokneam (IL)
(72) Inventor: Kornblau, Giora, 30500, Binyamina (IL); Maier Neustadter, David, 99785, Doar-Na Shimshon (IL); Shchory, Tal, 19245, Kibbutz Ramot Menashe (IL); Stokar, Saul, 43213, RaAnana (IL)
(74) Representative: Becker Kurig Straus

(57) **Abstract**

System and methods for image guided surgery is provided. The surgical tracking system (100) can include at least one sensor (134) configured to acquire a marker (104) signal associated with a first location of a marker within a body and to output a first signal indicative of the first location, and also further configured to acquire a device signal associated with a second location of a movable imaging device (114) and to output a second signal indicative of the second location. The movable imaging device can be configured to generate a plurality of sequential images. The surgical tracking system can further include at least one processor (210) configured to receive the first signal, the second signal, and the plurality of sequential images, and to generate a plurality of composite images where at least one of the plurality of composite images include a visual indication of the first location on at least one of the plurality of sequential images.

## Description

This application claims priority to U.S. Provisional Application No. 61/247,607, filed October 1, 2009, the contents of which are incorporated herein by reference. This application further claims priority to U.S. Provisional Application No. 61/333,519, filed May 11, 2010, the contents of which are incorporated herein by reference.

### Related Applications

This application is related to the following patent applications:

U.S. patent application 10/599,963, published as US2007/0205373, a U.S. National Phase application of PCT application PCT/IL2005/000871, filed on August 11, 2005 and published as WO06/016368, which takes priority from U.S. provisional applications 60/600,725, filed on August 12, 2004, and 60/619,897, filed on October 19, 2004;

U.S. patent application 11/463,664 filed on August 10, 2006 and published as US2007/0055144, a continuation-in-part of PCT application PGT/IL2005/000871;

U.S. patent application 11/990,315, published as US2009/0127459, a U.S. National Phase application of PCT application PCT/IB2006/052770, filed on August 10, 2006 and published as WO2007/017846, which takes priority from U.S. provisional application 60/773,930, filed on February 16, 2006;

U.S. patent application 11/463,659, filed on August 10, 2006 and published as US2007/0055090, and PCT application PCT/IB2006/052771, with the same title and filed on the same day, and published as WO2007/017847, which take priority from U.S. provisional application 60/773,931, filed on February 16, 2006;

U.S. patent application 11/665,844, published as US2008/0262473, a U.S. National Phase application of PCT application PCT/IL2005/001101, filed on October 19, 2005 and published as WO2006/043276, which takes priority from U.S. provisional applications 60/619,792 and 60/619,898, both filed on October 19, 2004;

U.S. patent application 11/791,890, published as US2009/0131734, a U.S. National Phase application of PCT application PCT/IL2007/000214, filed on February 15, 2007 and published as WO2007/094001, which takes priority from U.S. provisional applications 60/773,931, filed on February 16, 2006, and 60/804,178, filed June 28, 2006.

The contents of all of the above documents are incorporated by reference as if fully set forth herein.

### Field

The present disclosure, in some embodiments thereof, relates generally to systems and methods for guiding surgery using an implanted wireless marker, and a display apparatus that can indicate the location of the wireless marker and optionally can indicate the location of other predetermined intra-body structures or surfaces. More particularly, but not exclusively, in some embodiments, the wireless marker can be radioactive. Further still, the present disclosure, in some embodiments, relates generally to systems and methods for guiding surgery using a movable imaging device, where the locations of both the wireless marker and the movable imaging device can be tracked, and where the display apparatus can provide both the images from the movable imaging device and an indication of the location of the wireless marker, and optionally an indication of the location of other predetermined intra-body structures or surfaces.

### Background

Endoscopic surgery has become common practice in a number of medical fields including cardiology, urology, neurology, gastroenterology, gynecology, and oncology. During endoscopic, or other minimally invasive, procedures an imaging device, typically a camera and a light integral to the endoscope, is inserted into the patient's body. The imaging device can transmit an image of the organ or internal object undergoing the surgical procedure/inspection to the surgeon. The image can be used for diagnosis and/or to guide surgical tools during an operation.

Various endoscopes have been designed to meet the needs of specific surgical procedures. For example, the laparoscope is used to examine the interior of the abdomen and the thoracoscope is used to examine the interior of the chest. During video-assisted thoracoscopic surgery (VATS), a video imaging device can be inserted into the patient's chest and the thoracic surgeon can use the video images taken of the patient's internal anatomy during the operation to guide surgical instruments.

### SUMMARY

In one aspect, the present disclosure is directed to a surgical tracking system. The system can include at least one sensor adapted to acquire a marker signal associated with a first location of a marker within a body and to output a first signal indicative of the first location. The at least one sensor can also be adapted to acquire a device signal associated with a second location of a movable imaging device and to output a second signal indicative of the second location, the movable imaging device being configured to generate a plurality of sequential images. The surgical tracking system can also include at least one processor configured to receive data associated with the first signal, data associated with the second signal, and data associated with the plurality of sequential images, to generate data associated with a plurality of composite images, where at least one of the plurality of composite images include a visual indication of the first location on at least one of the plurality of sequential images.

An additional aspect of the present disclosure is directed to a method of guided surgery. The method can include implanting a marker inside a body and tracking a first location of the marker using a first sensor to acquire a marker signal associated with the first location of the marker. The method can further include acquiring an image of at least a portion of the body using an imaging device and tracking a second location of the imaging device using a second sensor to acquire a device signal associated with the second location of the imaging device. The method can further include displaying a composite image including the image of at least a portion of the body and an indication of a marker location.

An additional aspect of the present disclosure is directed to a surgical tracking system including a first sensor adapted to acquire a marker signal associated with a first location of a marker within a body and to output a first signal indicative of the first location. The system can further include a second sensor adapted to acquire a device signal associated with a second location of a surgical tool proximal to the body and to output a second signal indicative of the second location. The system can also include at least one processor configured to receive data associated with the first signal and data associated with the second signal, to compute at least one coordinate transformation between a first set of coordinates associated with the first location of the marker and a second set of coordinates associated with the second location of the surgical tool. The processor can be further configured to generate data associated with an indication of the location of the surgical tool with respect to the marker.

An additional aspect of the present disclosure is directed to a surgical tracking system. The system can include at least one sensor adapted to acquire a marker signal associated with a first location of a marker within a body and to output a first signal indicative of the first location. The at least one sensor can be further adapted to acquire a device signal associated with a second location of a movable display apparatus and to output a second signal indicative of the second location. The movable display apparatus device can be configured to generate a plurality of sequential data sets, each of the plurality of sequential data sets associated with a point of view of the movable display apparatus. The system can further include at least one processor configured to receive data associated with the first signal, data associated with the second signal, and data associated with the plurality of sequential data sets, to generate data associated with a plurality of projected indications, where at least one of the plurality of projected indications include a visual indication of the first location associated with at least one of the points of view of the movable display apparatus.

An additional aspect of the present disclosure is directed to a computer-readable medium storing a program for causing a computer to execute a method of guided surgery. The method can include implanting a marker inside a body and tracking a first location of the marker using a first sensor to acquire a marker signal associated with the first location of the marker. The method can also include acquiring an image of at least a portion of the body using an imaging device and tracking a second location of the imaging device using a second sensor to acquire a device signal associated with the second location of the imaging device. The method can further include displaying a composite image including the image of at least a portion of the body and an indication of a marker location.

Additional features and embodiments of the invention will be set forth in part in the description which follows, and in part will be obvious from the description, or may be learned by practice of the invention. It is to be understood that both the foregoing general description and the following detailed description are exemplary and explanatory only and are not restrictive of the claimed subject matter.

### BRIEF DESCRIPTION OF THE DRAWINGS

The accompanying drawings, which are incorporated in and constitute a part of this specification, illustrate exemplary embodiments of the disclosure and together with the description, serve to explain the principles of the disclosure. In the drawings,

FIG. 1 is a schematic diagram showing a guided surgery system using an external imaging device, consistent with an exemplary embodiment of the present disclosure;

FIG. 2 is a schematic diagram of a data processing system, consistent with the exemplary disclosed embodiment shown in FIGS. 1, 3, 4, and 5;

FIG. 3 is a schematic diagram showing a guided surgery system using an intra-body imaging device, consistent with an exemplary embodiment of the present disclosure;

FIG. 4 is a schematic diagram showing a guided surgery system using a tool, consistent with an exemplary embodiment of the present disclosure;

FIG. 5 is a schematic diagram showing a guided surgery system using a display apparatus, consistent with an exemplary embodiment of the present disclosure;

FIG. 6 is a flowchart for a guided surgery procedure using a system such as that shown in FIGS. 1, 3, 4, and 5;

FIG 7 schematically shows an image depicting the position of a tracked marker and other specified structures and boundaries; and

FIG 8 schematically shows use of an optional stereoscopic imaging system, with independent overlays to provide depth information.

### DESCRIPTION OF THE EMBODIMENTS

Reference will now be made in detail to the present exemplary embodiments, an example of which is illustrated in the accompanying drawings. Wherever possible, the same reference numbers will be used throughout the drawings to refer to the same or like parts.

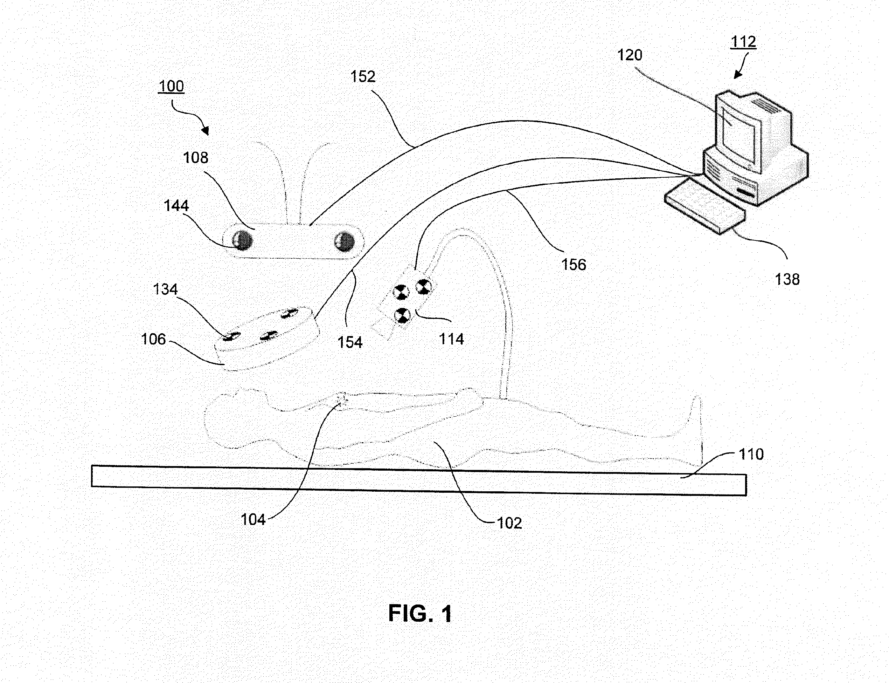

Referring now to the drawings, FIG. 1 schematically shows a guided surgery system 100 according to an exemplary embodiment consistent with the disclosure, used for performing surgery on a patient 102. In an alternative embodiment, guided surgery system 100 can be configured for performing exploratory or otherwise investigatory procedures on the internal organs or tissues of a patient 102. A wireless marker 104 can be implanted in the patient 102 before the surgery, for example to mark the position of a target tissue.

The wireless marker 104 is optionally constructed of a biocompatible material and can be configured to remain substantially in the place where it is implanted in the patient 102. The wireless marker 104 can be a marker that transmits a marking signal spontaneously (e.g., an energetic source of marking signals such as a radioactive marker), and/or a marker that transmits a marking signal in response to an externally applied field or signal (e.g., a marker that transmits a radio frequency signal in response to an externally applied field or signal), and/or a marker that reflects externally applied signals, such as an ultrasound or radar reflector. For example, the wireless marker 104 can be a radioactive marker comprising a material that emits gamma rays, which readily penetrate the body of the patient 102 and can be detected outside the patient 102. Exemplary radioactive markers for use as wireless marker 104 are described in U.S. Patent Application 11/791,890 (U.S. Patent Publication No. 2009/0131734), the contents of which have been incorporated herein-above by reference.

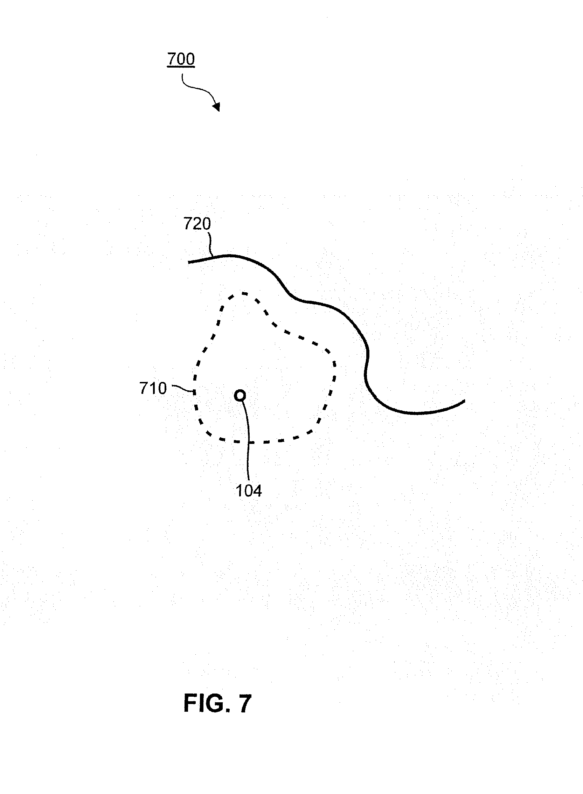

Optionally, the position of the wireless marker 104 relative to an isocenter of the target tissue (e.g., a tumor), for example, can be determined. If the wireless marker 104 is implanted into the target tissue, the offset from the center of the target tissue can be measured on pre-acquired images or on images acquired during the surgical procedure. For example, images can be acquired using conventional medical imaging methodologies, such as, computed tomography (CT) imaging, magnetic resonance imaging (MRI), x-ray imaging, or ultrasound imaging. Referring to FIG 7, in addition to showing the position of wireless marker 104, a pre-specified margin 710 of the target tissue (e.g., a tumor) can be specified. The margin 710, for example, can be the border of the tumor to be excised. If a portion of the margin 710 is specified on a stack of 2D images of the tumor, a 3D model of the margin 710 can be constructed. Optionally, in addition, other pre-specified structures of interest to the surgeon can be specified, such as a border 720 associated with critical structures (for example, blood vessels, nerves, or critical tissues and organs) that the surgeon may desire to avoid or bypass. Once again, if the border 720 of the critical structures is specified on a stack of 2D images, a 3D model of the border 720 associated with the critical structure can be constructed.

Referring back to FIG 1 of the drawings, a first tracking system 106 can be configured track the position of the wireless marker 104. The first tracking system 106 can include at least one sensor 134 configured to determine a direction associated with the wireless marker 104. For example, the sensor 134 can be configured to detect a signal emitted from the wireless marker 104 indicative of the relative direction between the sensor 134 and the wireless marker 104.

When the wireless marker 104 is a marker that transmits a marking signal spontaneously, or without the aid of an external field or signal such as a radioactive marker, the sensor 134 can be configured to detect the signal (e.g., gamma radiation) emitted by the wireless marker 104 and to generate a signal or signals indicative of an angle offset between a plane defined by the sensor 134, and in a known relationship to the first tracking system 106, and a plane occupied by the wireless marker 104. Exemplary sensors and tracking systems that are suitable for radioactive markers are disclosed, for example, in U.S. Patent Application Nos. 10/599,963 (U.S. Patent Publication No. 2007/0205373) and 11/990,315 (U.S. Patent Publication No. 2009/0127459), the contents of which have been incorporated herein-above by reference..

Alternatively, or in addition, the wireless marker 104 can be a marker that transmits and/or reflects a marking signal with the aid of, or in response to, an externally applied field or signal (such as a marker that transmits a radio frequency signal in response to an external magnetic field or electromagnetic field, or in response to another radio frequency signal, and/or a marker that reflects externally applied signals, such as an ultrasound or radar reflector). The sensor 134 can again be configured to detect a signal emitted by the wireless marker 104 and to generate a signal or signals indicative of the relative distance between the sensor 134 and the wireless marker 104 (e.g., the radius of the three-dimensional sphere surrounding the sensor 134 on which the wireless marker 104 resides). Sensors and systems suitable for RF markers are described, for example, in U.S. Patent Publication No. 2005/0059884 and U.S. Patent Publication No. 2002/0193685, both assigned to Calypso Medical, Inc.

The first tracking system 106 can be free to move and/or to be oriented in different directions, relative to the patient 102. For example, in an embodiment, the first tracking system 106 can track the location of the wireless marker 104 relative to an internal coordinate system (CS₁₀₆) of the first tracking system 106. For example, as described above in one embodiment, the sensor 134 can be a radioactive tracking sensor and the signal emitted from the marker 104 can be a gamma radiation signal. The sensor 134 can be configured to determine a direction associated with the source of the gamma radiation signal. Alternatively, or in addition, sensor 134 can be an RF tracking sensor, and the signal emitted from the wireless marker 104 can be a radio frequency signal. The sensor 134 can be configured to determine the radius of a sphere on which the wireless marker 104 resides. Where the first tracking system 106 utilizes a plurality of sensors 134, then the first tracking system 106 can be configured to determine a three-dimensional location of the wireless marker 104 expressed in terms of the coordinate system CS₁₀₆ of the first tracking system 106.

In an embodiment, the position and/or orientation of the first tracking system 106 can itself tracked by a second tracking system 108. The second tracking system 108 can be (but is not required to be) fixed with respect to the patient 102, or can be fixed with respect to a surgical bed 110. Optionally, the first tracking system 106 can include an inertial tracking system so that its position and/or orientation can be tracked relative to its initial position and/or orientation. Optionally, there can be a direct line of sight between the first tracking system 106 and the second tracking system 108, and the second tracking system 108 can utilize optical tracking. Alternatively or additionally, the second tracking system 108 can use one or more other tracking methods, such as RF or magnetic tracking, or radioactive tracking, which may not require a direct line of sight. The second tracking system 108 can be configured to track the location of the tracking system 106 relative to an internal coordinate system (CS₁₀₈) of the second tracking system 108.

The tracking data from the first tracking system 106, and the second tracking system 108, can be processed by a processor 210, which can be associated with a data processing system 112, and which can be a workstation or other computing system or a dedicated chip or chips. The processor 210 can be configured to receive the data associated with the location of the wireless marker 104 in the internal coordinate system CS₁₀₆ of the first tracking system 106, and to calculate the location of the wireless marker 104 with respect to the coordinate system CS₁₀₈ of the second tracking system 108, or (where relevant) another external coordinate system, such as a coordinate system fixed with respect to surgical bed 110, or a coordinate system that may be fixed with another movable device, such as a display apparatus, including, without limitation, a heads-up display and or movable projection device as described further below.

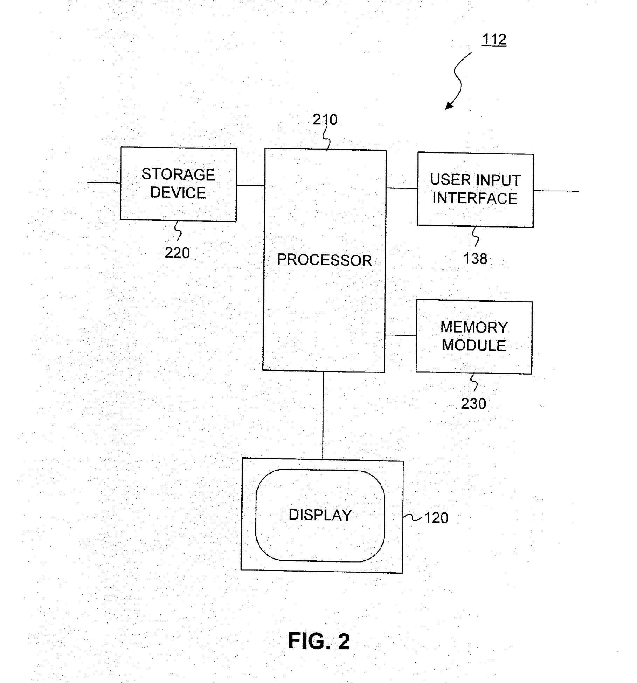

Referring to FIG. 2, the data processing system 112 can be associated with one or more software applications, including, for example, an image processing application, a coordinate transform application (capable of transforming coordinates from a first coordinate system to a second coordinate system), and/or a statistics application related to gamma-ray counting. These software applications can be stored on the data processing system 112, and can be accessed by a user in a guided surgery environment. The software applications also can be stored on a computer readable medium, such as a hard drive, computer disk, CD-ROM, or any other suitable medium. The data processing system 112 can be coupled to the first tracking system 106, the second tracking system 108, the movable imaging device 114, and other system components using a wireless connection, for example, a cloud networked application. Data received by each of the system components can be transferred to the data processing system 112.

FIG. 2 is a schematic diagram of the data processing system 112. The data processing system 112 can include a processor 210, a memory module 230, a storage device 220, an input/output interface 138, and a display device 120. The data processing system 112 can include additional, fewer, and/or different components than those listed above. The type and number of listed devices are exemplary only and not intended to be limiting.

The processor 210 can be a central processing unit ("CPU") or a graphic processing unit ("GPU"). The processor 210 can execute sequences of computer program instructions to perform various processes that will be explained in greater detail below. The memory module 230 can include, among other things, a random access memory ("RAM") and a read-only memory ("ROM"). The computer program instructions can be accessed and read from the ROM, or any other suitable memory location, and loaded into the RAM for execution by the processor 210. Depending on the type of the data processing system 112 being used, the processor 210 can include one or more printed circuit boards, and/or one or more microprocessor chips.

The storage device 220 can include any type of mass storage suitable for storing information. For example, the storage device 220 can include one or more hard disk devices, optical disk devices, or any other storage devices that provide data storage space. In one embodiment of the present disclosure, the storage device 220 can store data related to the data processing process, such as image data received, and/or tracking data received, and any intermediate data created during the data processing process. The storage device 220 can also include analysis and organization tools for analyzing and organizing the information contained therein.

The data processing system 112 can be accessed and controlled by a user, such as a surgeon or a surgeon's assistant, using input/output interface 138. User input/output interface 138 can be provided for the user to input information into data processing system 112, and can include, for example, a keyboard, a mouse, touch screen, and/or optical or wireless computer input devices. The user can input parameters to adjust the operation of the data processing system 112. Optionally, the user can input and store notes and any other data relevant to guided surgery system 100 or the patient 102. In an embodiment, using the data processing system 112, the user can adjust or otherwise modify the function and/or location/orientation of the first tracking system 106, the second tracking system 108, and the movable imaging device 114.

The data processing system 112 can also provide visualized information via the display device 120. For example, the display device 120 can include a computer screen and can provide a graphical user interface ("GUI") to the user. Consistent with one embodiment, the display device 120 can display an image of a portion of the patient 102 and an indication of the position of the wireless tracker 104. It is contemplated that the composite image can be updated in real time as the data corresponding to the location of the marker 104 and the patient image is updated.

It can further be noted that, whereas it may be particularly advantageous for the first tracking system 106 to use radioactive tracking to track the wireless marker 104, because the wireless marker 104 can be located inside the body of the patient 102 and can be very small, it may not be advantageous for the second tracking system 108 to use radioactive tracking to track the first tracking system 106, particularly if the first tracking system 106 is relatively large and is located outside the body of the patient 102. Instead, it may be more convenient for the second tracking system 108 to use optical, magnetic, or RF tracking to track the first tracking system 106.

In an embodiment, the guided surgery system 100 can include a movable imaging device 114, which can obtain imaging data associated with a view of the patient 102. The movable imaging device 114 can be an extra-body imaging device. The orientation of the images captured by the movable imaging device 114 can be oriented relative to the internal coordinate system (CS₁₁₄) of the movable imaging device 114 and can be converted to the coordinate system CS₁₀₈ of the second tracking system 108, or (where relevant) another external coordinate system, such as a coordinate system fixed with respect to surgical bed 110, or a coordinate system that may be fixed with another movable device, such as a movable display apparatus, including, without limitation, a heads-up display, see-through goggles, and/or a movable projection device as described further below.

In the embodiment shown in FIG. 1, the movable imaging device 114 can be an external imaging device, such as an optical or infrared camera, an x-ray detector for ordinary x-ray imaging or CT imaging, an ultrasound detector, or an MRI receiver. Movable imaging device 114 can be moveable and/or rotatable, and optionally the second tracking system 108, or a separate imager tracking system, can track the position and/or orientation of the movable imaging device 114. In a further embodiment, such tracking can be with respect to the same coordinate system (such as a coordinate system fixed with respect to the surgical bed 110) that can be used for tracking the wireless marker 104. Imaging data from the movable imaging device 114, and any tracking data associated with the position and/or orientation of the movable imaging device 114, can be processed by the processor 210, or a separate processor or processors, to reconstruct an image of at least part of the patient 102, registered to a known position and orientation with respect to such a fixed coordinate system. The processor 210, or a separate processor or processors, can synthesize a combined image on the display device 120, including an indication of the location of the wireless marker 104 superimposed on an image of a portion of the patient 102 at an appropriate location. The indication of the location of the wireless marker 104 can be displayed, for example, as a symbol, such as a dot or an icon of a different type, and optionally displayed in a color that will make it stand out from the image of the patient 102.

In an embodiment, the movable imaging device114 can be a two-dimensional imaging device. The two-dimensional representation of the margin 710 and the border 720 of critical structures, can be overlaid with the image capured by the movable imaging device 114 and can be displayed to the user via the display device 120. Optionally, if the offset of the wireless marker from the target isocenter has been measured, the marked position can be the target isocenter. Optionally, if the margin 710 has been specified, the isocenter and/or the boundary (e.g., the margin 710) can be displayed on the image in a distinguishable manner. Optionally, if additional structures (e.g., the critical structures) have been predetermined, their positions (e.g., border 720) can also be indicated on the image of the patient 102 in a distinguishable manner. Optionally, the combined image synthesized by the processor 210 can be an image of the wireless marker 104, acquired in real time, together with the image of the patient 102, superimposed on the image of the patient 102.

In another embodiment, the movable imaging device 114 is a three-dimensional imaging device, and a three-dimensional representation of the margin 710 and the border 720 can be overlaid on to the coordinating three-dimensional image of the marker 104. In an embodiment, the movable imaging device 114 can be a stereoscopic imaging device, the display device 120 can show various stereoscopic views of the margin 710 and the border 720 using pairs of images extracted from the three-dimensional data set acquired using conventional medical imaging.

As described herein, guided surgery system 100 can provided a visual display including a symbol indicative of the location of the wireless marker 104 and that can comprise pictorial representations of the wireless marker 104, and optionally any of the additional overlays such as the margin 710 and/or the border 720, stored previously in storage device 220 and available to processor 210.

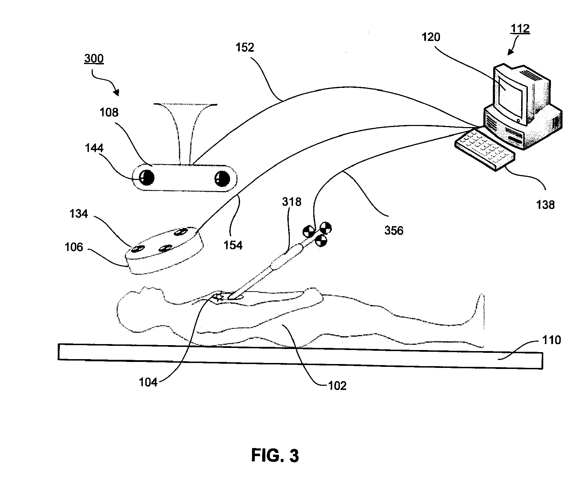

FIG. 3 shows a guided surgery system 300 similar to the guided surgery system 100, but with a movable imaging device 318 that is an intra-body imaging device, such as an endoscope, instead of the movable imaging device 114 corresponding to an external imaging device. Exemplary embodiments of an intra-body movable imaging device 318 include endoscopes such as, for example, a laparoscope used in abdominal or pelvic cavity procedures, and a thoracoscope used in chest cavity procedures. Where the movable imaging device 318 is an intra-body system, the image generated can include internal tissue and structures proximate the movable imaging device 318 within the patient 102. Optionally, an array of fiber optic cables in the movable imaging device 318 can be used to convey an image produced inside the body of the patient 102 to a detector array located outside the body. The position and/or orientation of the movable imaging device 318, similar to the position and/or orientation of the movable imaging device 114 in FIG. 1, is optionally tracked by the second tracking system 108, or by a separate tracking system. Optionally, the separate tracking system can use a different tracking technology than that employed by the second tracking system 108. Optionally, the tracking is done using a portion of the movable imaging device 318 that is outside the body of the patient 102, and has a line of sight to the second tracking system 108, for example using optical tracking. Alternatively or additionally, the tracking can be done using a portion of the movable imaging device 318 that is inside the body or can be inside the body, for example using RF tracking or electromagnetic tracking. Alternatively, the external part of the movable imaging device 318 can be tracked using one tracking system, while the position of the tip of the movable imaging device 318 relative to its body can be tracked using a second tracking system, such as a set of strain gauges or an optical system for tracking flexible optical cables.

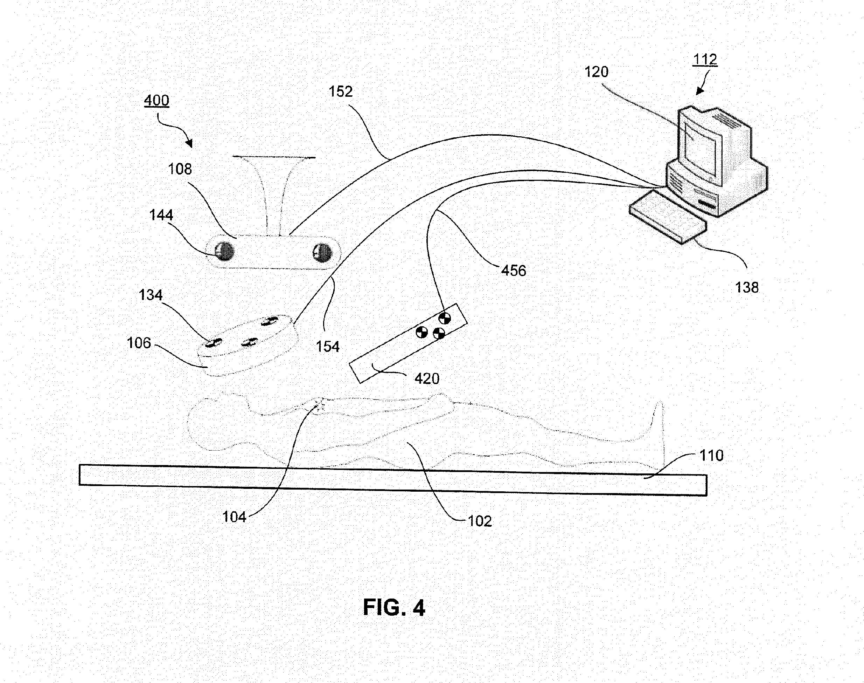

FIG. 4 shows a guided surgery system 400 similar to the guided surgery system 100 and the guided surgery system 300, but with a tool 420, instead of the movable imaging device 114 and the movable imaging device 318, corresponding to an external imaging device and an intra-body imaging device, respectively. The tool 420 can be any apparatus used during a surgical procedure either inside or outside the body of the patient 102. The position and/or orientation of the tool 420, similar to the position and/or orientation of the movable imaging device 114 in FIG. 1 and the movable imaging device 318 in FIG. 3, is optionally tracked by the second tracking system 108, or by a separate tool tracking system. Optionally, the separate tracking system can use a different tracking technology than that employed by the second tracking system 108. Optionally, the tracking is done using a portion of the tool 420 that is outside the patient's body, and has a line of sight to the second tracking system 108, for example using optical tracking. Alternatively or additionally, the tracking can be done using a portion of the tool 420 that is inside the body or can be inside the body, for example using RF tracking or electromagnetic tracking. Alternatively, the external part of the tool 420 can be tracked using one tracking system, while the position of the tip of the tool 420 relative to its body can be tracked using a second tracking system, such as a set of strain gauges or an optical system for tracking flexible optical cables.

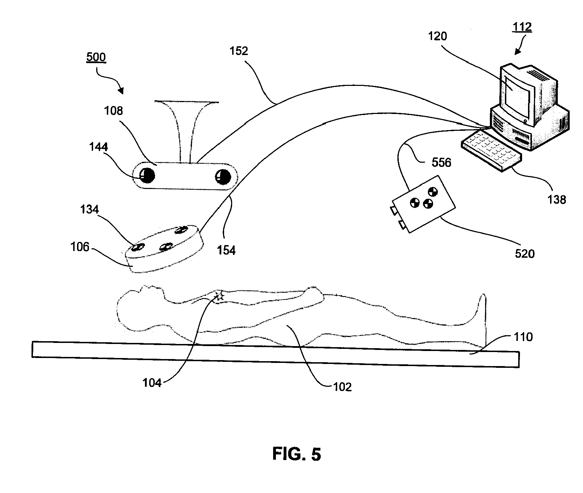

Referring to FIG. 5, and in some embodiments consistent with the disclosure, the guided surgery system 500 can be configured to project information onto the body of the patient 102. Exemplary information can include any graphic or textual information helpful to the user such as, for example, a projected indication of the location of the wireless marker 104 onto the body of the patient 102, for example onto the skin of the patient 102 adjacent to the location of the wireless marker 104. In an embodiment, the guided surgery system 100, 300, 400, and/or 500 can also be configured to superimpose an indication of the location of marker 104 on the image depicted on the display device 120. For example, FIG. 5 shows a guided surgery system 500 similar to the guided surgery system 100, the guided surgery system 300, and the guided surgery system 400, but with a display apparatus 520. In one embodiment, display apparatus 520 can be a movable projection device that is configured to project an indication on a surface proximal to the wireless tracker 104, such as on the skin or other exposed portion of the patient 102. In a further embodiment, display apparatus 520 can be a heads-up display or special see-through goggles or eyeglasses configured to display an image to the wearer of the device. The position and/or orientation of the display apparatus 520, similar to the position and/or orientation of the movable imaging device 114 in FIG. 1, the movable imagine device 318 of FIG. 3, and the tool 420 of FIG. 4, is optionally tracked by the second tracking system 108, or by a separate display tracking system. Optionally, the separate tracking system can use a different tracking technology than that employed by the second tracking system 108. Optionally, the tracking is done using a portion of the display apparatus 520 that has a line of sight to the second tracking system 108, for example using optical tracking. Alternatively or additionally, the tracking can be done, for example using RF tracking or electromagnetic tracking.

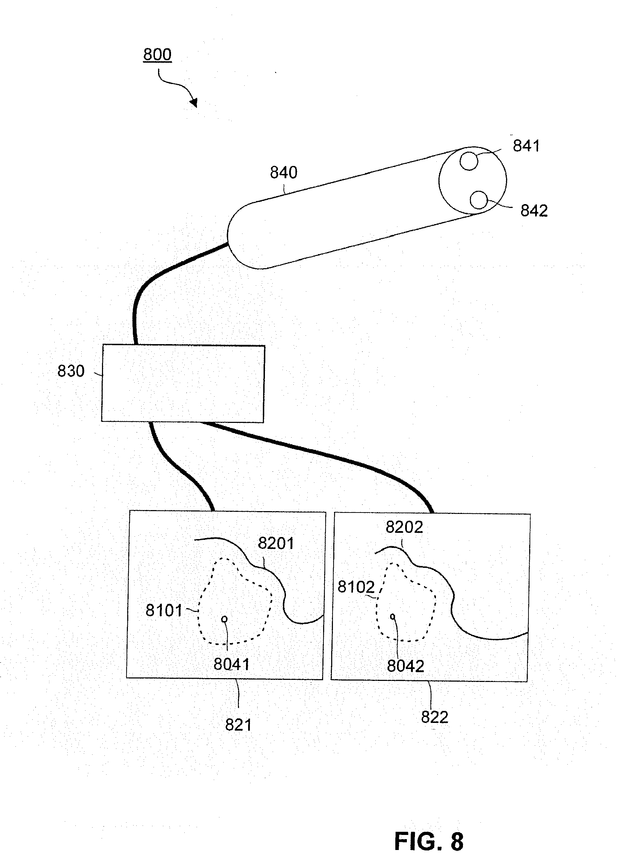

As described above, in some embodiments consistent with the disclosure, instead of or in addition to displaying an image on the display device 120, an image can be displayed using special see-through goggles or eyeglasses. Such display techniques are well known in the field of heads-up displays for use by fighter pilots, as well as for augmented reality or enhanced reality systems. Optionally, the see-through goggles or eyeglasses display only an indication of the location of the wireless marker 104, superimposed on a real view of the patient seen through goggles or eyeglasses. For such a display, the second tracking system 108, or a separating tracking system, optionally tracks the position and orientation of the goggles or eyeglasses, or of the head of the person wearing them, so that the indication of the location of the wirleless marker 104 will appear in the right location in the display. Optionally, the correct location in the display, for the indication of the location of the wireless marker 104, can be calculated separately for the left and right eyes, so that a stereoscopic effect indicates to the surgeon a full three-dimension location of the wireless marker 104, including its depth inside the body of the patient 102. Referring to FIG. 8, which depicts the use of a stereoscopic laparoscope, the stereoscopic camera 840 obtains images using two individual cameras (841 and 842) which can correspond to a "left" view and a "right" view respectively. A processing unit 830 can be used to calculate and/or generate separate images for each eye, with overlays showing the position of the wireless marker 104 (reference numbers 8041 and 8042), the margin 710 (reference numbers 8101 and 8102), and the border 720 of a critical structure (reference numbers 8201 and 8202), where separate images are generated and displayed for the left and right eyes (image 821 and image 822 respectively). Optionally, the display imaging system can be a 3D imaging system, so that depth can be displayed without a stereoscope.

The guided surgery system 100, 300, 400, and 500 can be used for both endoscopic and traditional surgical procedures. In various embodiments consistent with the disclosure, the indication of the location of the wireless marker 104 can be shown with respect to features of the patient 102 accessible to the surgeon, for example the outer surface of the body of the patient 102. The indication of the location of the wireless marker 104 can be used by the surgeon in guiding a surgery, to help locate the wireless marker 104 and the tissue adjacent to the wireless marker 104, which can include a suspected tumor, for example. The indication of location of the wireless marker 104 can optionally include an indication of the depth of the wireless marker 104 under the surface of the body of the patient 102, for example using a stereoscopic or holographic display, or using a symbolic indication of depth, such as color-coding, or a degree of fuzziness, or displaying a number indicating the depth.

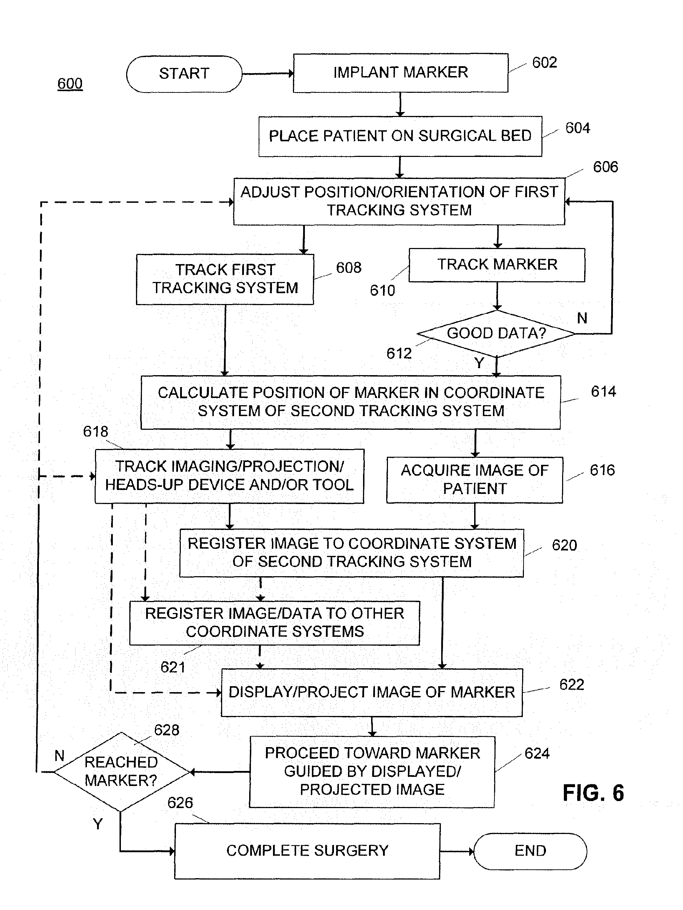

FIG. 6 depicts a flowchart 600, for performing a guided surgery method using a system such as the guided surgery system 100 in FIG. 1, or the guided surgery system 300 in FIG. 3, the guided surgery system 400 in FIG. 4, the guided surgery system 500 in FIG. 5, and/or any of the guided surgery systems 100, 300, 400, 500 optionally using the heads-up and/or stereoscopic devices consistent with FIG. 8. The wireless marker 104 can be implanted at a position inside the patient 102 at Step 602, some time before surgery. For example, in the case of thoracoscopic surgery to find and remove a pulmonary tumor or nodule associated with the patient 102, the wireless marker 104 can be attached to the tumor or nodule, or to tissue nearby, optionally using ultrasound guidance to inject and attach it at an appropriate position. In the case of surgery to remove a breast tumor or cyst from the patient 102, the wireless marker 104 can be injected into or near the tumor or cyst under ultrasound guidance. Optionally, before the surgical procedure is begun, or during the procedure, the offset of the wireless marker 104 from or to the isocenter of the target tissue can be measured. Optionally, the margin 710 associated with the target to be removed can be specified as well. Optionally, the positions and/or borders 720 associated with other critical structures can be specified.

At Step 604, the patient 102 can be placed on a surgical bed 110, such as is depicted in FIGS. 1, 3, 4, and 5, and which can be held at a fixed location relative to the first tracking system 106 or additional tracking systems, such as the second tracking system 108 in FIGS. 1, 3, 4, and 5. The second tracking system 108 can be used to indirectly to track the wireless marker 104, and can be used to directly track the movable imaging device 114, the movable imaging device 318, the tool 420, the display apparatus 520 (such as a movable projection device, any heads-up device 800, and/or see-through goggles) with respect to the internal coordinate system CS₁₀₈ of the second tracking system 108. Optionally, the patient 102 can be constrained to be at a fixed location and posture with respect to the surgical bed 110, during the surgery. Optionally, whether or not the surgical bed 110 and the patient 102 are held fixed, there can be a common coordinate system (e.g., the internal coordinate system CS₁₀₈ of the second tracking system 108) that both the movable imaging device 114, the movable imaging device 318, the tool 420, and/or the movable display apparatus 520 and the first tracking system 106 for the wireless marker 104 are referenced to.

Optionally, the first tracking system 106 can be movable and can be adjusted in position and/or orientation at Step 606. The position and orientation may be chosen, for example, so that the tracking sensors 134 in the first tracking system 106 are close enough to the wireless marker 104 to provide adequate precision in locating and/or tracking the wireless marker 104, but are not so close that they get in the way of the surgeon. In an embodiment, the tracking sensors 134 can detect a signal emitted from the wireless marker 104 in three dimensions, and can be configured to generate signals associated with the relative direction between the sensor 134 and the wireless marker 104 and/or the distance between sensor 134 and the wireless marker 104. In an embodiment, the sensors 134 can be mounted on the first tracking system 106, and the sensors 134 can be aimed towards the patient 102. In an exemplary embodiment, the sensors 134 can be located on a single surface of the first tracking system 106. In another exemplary embodiment, first tracking system 106 can include multiple sensors 134 located on multiple surfaces of the first tracking system 106.

The position and orientation of the first tracking system 106 can vary depending on the position and orientation of the patient 102, on the location of the wireless marker 104 within the body of the patient 102, and in response to user input/instructions. The position and orientation of the first tracking system 106 can also change as the surgery proceeds, for example to stay out of the way of the surgeon if he or she works from a changed position or direction.

At Step 610, the wireless marker 104 can be tracked by the first tracking system 106 with respect to the internal coordinate system CS₁₀₆ of the first tracking system 106. As described above at Step 608, the first tracking system 106 can be tracked by the second tracking system 108 with respect to the internal coordinate system CS₁₀₈ of the second tracking system 108, as it can move in real time during the surgery. Optionally, the first tracking system 106 and the second tracking system 108 can operate substantially simultaneously, as indicated in flowchart 600. Alternatively, the position and/or orientation of the first tracking system 106 can be measured by the second tracking system 108 before or after the first tracking system 106 determines or measures the location of the wireless marker 104, but where the first tracking system 106 does not necessarily move very much in that time. Optionally, the position and/or orientation of the first tracking system 106 is not measured or acquired until it is known that the first tracking system 106 has obtained good data for tracking the wireless marker 104.

Optionally, at Step 612, a determination is made as to whether the tracking data associated with the wireless marker 104 is suitable to provide adequate precision for determination a location of the wireless marker 104, and if not, the position and/or orientation of the first tracking system 106 can be adjusted again at Step 606. For example, the first tracking system 106 can be brought closer to the part of the body of the patient 102 where the wireless marker 104 is located, and/or the first tracking system 106 can be rotated so that its direction of greatest angular sensitivity is directed more closely toward the wireless marker 104. Adequate precision is, for example, within 2 centimeters, or 1 centimeter, or 5 mm, or 2 mm, or 1 mm, with, for example, a 90% confidence level. The precision of the first tracking system 106 can be determined from the tracking data, for example, by a theoretical modeling of the tracking process, and/or by empirical studies relating characteristics of the tracking data to the precision of the tracking. This analysis can be carried out in connection with processor 210, or another processor or processors. In the case of radioactive tracking, for example, the precision can depend primarily on the statistics of gamma-ray counting, and can be calculated from the tracking data, using methods that are well understood by those skilled in the art of radioactive detectors, and statistics.

At Step 614, the position of the wireless marker 104 can be calculated, using the tracking data from the first tracking system 106 and the second tracking system 108, in reference to both the internal coordinate system CS₁₀₆ of the first tracking system 106 and the internal coordinate system CS₁₀₈ of the second tracking system 108.

At Step 616 an image of the patient 102 can be acquired, for those embodiments consistent with the disclosure in which a movable imaging device 114 and/or movable imaging device 318 acquires an image of the patient 102 in real time. In an embodiment, the movable imaging device 114 and/or the movable imaging device 318 can acquire a single image or a plurality of images. In an embodiment, the movable imaging device 114 and/or the movable imaging device 318 can capture a plurality of sequential images. At Step 618, where an image is acquired by the moveable imaging device 114 and/or the movable imaging device 318, the position and/or orientation of the movable imaging device 114 and/or 318 can be tracked. In addition, or alternatively, the position and/or orientation of the tool 420 and/or the movable display apparatus 520 (such as any movable projection device, any heads-up device 800, and/or see-through goggles) can be tracked. The position and/or orientation of the movable imaging device 114 and/or 318, the tool 420, and/or the movable display apparatus 520 can change in real time during surgery. For example, movable imaging device 114 and/or 318 can be pushed out of the way in order to avoid interfering with the actions of the surgeon. Or, in the case of surgery using an endoscope or similar instrument, the movable imaging device 318 can be comprised in the endoscope, and can move as the endoscope is moved through the patient 102 as part of the surgical procedure. These changes in the position and/or orientation of the movable imaging device 114 and/or 318, the tool 420, and/or the movable display apparatus 520 can be tracked at Step 618 by the second tracking system 108 or by a separate tracking system, such as an imager tracking system, and/or a movable projection device tracking system, and/or a heads-up display or see-through goggles tracking system. Tracking the movable imaging device 114 and/or 318, the tool 420, and/or the movable display apparatus 520 in real time has the potential advantage that it allows the registration of any acquired image with the internal coordinate system CS₁₀₈ of the second tracking system 108, or any other coordinate system to be updated in real time, so that a display of the acquired image, with a superimposed indication of the location of the wireless marker 104, can be updated in real time during the surgery.

Optionally, as indicated in flowchart 600, tracking the movable imaging device 114 and/or 318, the tool 420, and/or the movable display apparatus 520 at Step 618 can be done simultaneously with acquiring the image at Step 616. Alternatively, the position and/or orientation of the movable imaging device 114 and/or 318 can be determined before or after acquiring the image, but without the movable imaging device 114 and/or 318 moving very much between the time when its position and/or orientation are determined, and the time when the image is acquired. Optionally, acquiring the image and/or tracking the movable imaging device 114 and/or 318 can be done before, or simultaneously with, tracking the wireless marker 104 and/or tracking the first tracking system 106, rather than afterward as depicted in an exemplary manner in flowchart 600. In some embodiments consistent with the disclosure, the guided surgery system 100, 300, 400, and/or 500 uses one or more images of the patient 102, or generic images of a person, acquired previously. Consistent with the disclosure in such an embodiment, Steps 616 and 618 can be skipped.

The image of the patient 102 can be acquired using any known imaging modality, and can be a two-dimensional or three-dimensional image. For example, an optical or infrared camera can be used, or an ultrasound or MRI imaging system, or a regular x-ray or CT imaging system. In the case of an MRI imaging system, a fixed main magnet and fixed gradient coils can be used, as in conventional MRI, optionally with an open MRI system to facilitate access by the surgeon. In that case, even if a moveable MRI receiver antenna is used to detect the MRI signals, the position of the receiver antenna may or may not be important for reconstructing the image with respect to an external coordinate system, and if it is not important, it need not be tracked. Alternatively, a small self-contained MRI probe can be used, and in that case, if the MRI probe is moveable, it can be tracked in order to relate the acquired images to the external coordinate system.

For example, in the case of thoracoscopic surgery to remove a pulmonary tumor or nodule, the image of the patient 102 can be acquired with the thoracoscope, and the thoracoscope can be tracked, for example with an optical tracker recording the position of a visible part of the thoracoscope that is outside the body of the patient 102. Optionally, if the thoracoscope has degrees of freedom that allow a portion of it to twist and/or turn inside the body of the patient 102, where it is not visible to the optical tracker, then it can include sensors which record these degrees of freedom, so that the position and orientation of a distal end of the thoracoscope, which acquires an image, can be determined from the optical tracking data together with data from these sensors. Similar remarks apply to surgery performed or aided with any kind of endoscope or laparoscope, including robotic laparoscopic surgery, in which the endoscope or laparoscope produces an image of a part of the body of the patient 102 that is accessible to the surgeon.

In the case of surgery to remove a breast tumor or cyst, the image of the patient 102 can be acquired with an optical or infrared camera. The camera can be mounted on a configurable arm such as a gooseneck, allowing it to be moved to a position and orientation where it has a line of sight to the breast, without getting in the way of the surgeon or any member of the surgical team.

At Step 620, the acquired image can be registered to the internal coordinate system CS₁₀₈ of the second tracking system 108 using the tracking data obtained at Step 618. In addition, or alternatively, an acquired image can be registered to any other available coordinate system, such as the internal coordinate system that be associated with a heads-up display device 800. This is depicted at Step 621.

At Step 622, an indication of the location of the wireless marker 104 can be displayed. In some embodiments consistent with the disclosure, processor 210 can be configured to synthesize image data such that the display device 120 provides an image, comprising the acquired image and a superimposed indication of the location of the wireless marker 104. As indicated, the synthesis of the image data can be performed by a processor or processors, such as processor 210. The guided surgery system 100 and/or 300 can be configured to display the synthesized image, showing the indication of the location of the wireless marker 104, superimposed on the image of the patient. Optionally, if the offset of the wireless marker 104 from the target isocenter has been specified, the marked position can be the target isocenter. Optionally, if the margin 710 has been specified, both the isocenter and/or the boundary (e.g., the margin 710) can be displayed on the synthesized image in a distinguishable manner. Optionally, if additional structures have been predetermined (e.g., the boundary 720 of critical structures) their positions can be indicated on the image of the patient 102 in a distinguishable manner. Optionally, the movable display apparatus 520 (as a movable projection device) can project an indication of the location of the wireless marker 104 proximal to or on the body of the patient 102.

Solutions to the problem of indicating the position of a point whose coordinates are known in one coordinate system, on a image acquired in another coordinate system, are well known to those skilled in the art of image display and synthesis. For example, an exemplary method is described in U.S. patent 6,226,543, to Gilboa et al., at col. 14, lines 6-67 and col. 15 lines 1-11.

The image of the patient 102, on which the indication of the location of the wireless marker 104 is superimposed, can depict features of the patient 102 that are accessible to the surgeon, for example the field that the surgeon is working in, in the case of breast surgery with an external camera, or the view through the thoracoscope, in the case of thoracoscopic lung surgery. Optionally, the image can depict the direction of the marker from the point of view of the movable imaging device 114 and/or 318, even if the wireless marker 104 is hidden from view. Optionally, the margin 710 of the target or the boundary 720 of other critical structures may be within the field of view of the movable imaging device 114 and/or 318, even if the wireless marker 104 itself is hidden. The superimposed image that is displayed by the guided surgery system 100 and/or 300 can help to guide the surgeon in reaching the tissue marked by the wireless marker 104, while minimizing or reducing unnecessary trauma to the patient 102. In the case of a three-dimensional image, for example a CT or MRI image, the guided surgery system 100 and/or 300 can display the three-dimensional position of the wireless marker 104 in a perspective view, optionally giving a viewer of the display control over the point of view of the perspective view. Optionally, the perspective view shows only some body tissues, for example it is an angiography image showing only blood vessels, so that it is easier to visualize a large range of depths simultaneously. Alternatively or additionally, the guided surgery system 100 and/or 300 can display the position of the wireless marker 104 and optionally the target boundary (e.g., the margin 710) and optionally the boundary 720 of other predetermined structures in a two-dimensional slice of the three-dimensional image, optionally giving the viewer control over the position and/or orientation of the slice. The orientation of the two-dimensional slice in which the position of the wireless marker 104 can be displayed need not be the same as the orientation of two-dimensional slices from which the three-dimensional image was originally acquired.

In an optional embodiment consistent with the disclosure, the movable imaging device 114 and/or 318 used by the surgeon, such as the thoracoscope or laparoscope, can be a stereoscopic system, that is, it contains different images for each eye. In this case, the overlay of the wireless marker 104 position, pre-specified boundary (e.g., margins 710) and/or pre-specified tissues (e.g., critical structures and/or their boundaries 720) can be calculated separately for the image for each eye, giving the user improved depth perception.

In some embodiments consistent with the disclosure, instead of or in addition to displaying an image of the patient 102 with an indication of the location of the wireless marker 104 superimposed, an indication of the location of the wireless marker 104 can be projected directly onto the patient 102 by the projector 520. For example, and without limitation, a spot of light indicating the location of the wireless marker 104 can be projected onto the skin of the patient 102, close to the location of the wireless marker 104 inside the body of the patient 102, and this indication of the position of the wireless marker 104 can be used to guide the surgeon.

Many options for displaying the position of an intrabody marker (such as wireless marker 104 in some embodiments) on the surface of the patient 102 are given in U.S. patent 6,690,964 to Beiger et al., and optionally may be used. In particular, the position of the wireless marker 104 can be projected either with or without a correction for the line of sight of the surgeon. Optionally the surgeon's line of sight can be taken into account, and can be tracked as well. As noted above, the line of sight of the surgeon can also be tracked if the surgeon is wearing a heads up display that indicates the location of the wireless marker 104 on the surgeon's real view of the patient.

At Step 624, the surgeon can proceed towards the wireless marker 104, guided by the image displayed by the guided surgery system 100, 300, 400, 500, and/or any stereoscopic or heads-up device 800. At Step 628, if the surgeon has not yet reached the location of the wireless marker 104, the position and/or orientation of the first tracking system 106 can be optionally adjusted again at Step 606. Alternatively, the first tracking system 106 need not adjusted at this time, but the movable imaging device 114 and/or 318 can be moved, and its position and/orientation can be tracked at Step 618. This can be done, for example, if the movable imaging device 318 comprises an endoscope, attached to a laparoscopic surgical instrument which the surgeon is using to progress closer to the wireless marker 104. Alternatively, if the movable imaging device 114 comprises an external imaging system, and the surgery is a conventional open surgery, then the movable imaging device 114 can be moved to a new position or angle to get a better view of an incision made by the surgeon, as it moves closer to the wireless marker 104. Even if the first tracking system 106 and the movable imaging device 114 and/or 318 are not moved, their positions and/or orientations optionally continue to be tracked, to determine if they have moved or to verify that they have not moved.

Optionally, the time required for the first tracking system 106 to find the location of the wireless marker 104 to within a desired precision is sufficiently short, so that the wireless marker 104 can be tracked in real time, as its position may change due to disturbance of the body of the patient 102 during the surgical procedure. For example, locating the wireless marker 104 can take less than 1 second, or less than 2 seconds, or less than 5 seconds, or less than 0.5 seconds, or less than 0.2 seconds. Optionally, the time required for the full loop of the flowchart in FIG. 6, from Step 606 to Step 628 and back to Step 606, can also be less than 1 second, or less than 2 seconds, or less than 5 seconds, or less than 0.5 seconds, or less than 0.2 seconds, and the position of the wireless marker 104 can be updated at these intervals, or shorter intervals. Optionally, the acquired image is updated at the same intervals, or at longer or shorter intervals, than the position of the wireless marker 104. As noted above, the precision in the location of the wireless marker 104 can be 2 cm, 1 cm, 5 mm, 2 mm, or 1 mm, optionally with a 90% confidence level. Knowing the location of the wireless marker 104 to a relatively high precision can allow the surgeon to zero in on it while doing minimal damage to body tissues. Optionally, being shown the margins 710 of the tissue to be removed helps the surgeon be sure he or she has removed all of the target tissue without any additional tissue. Optionally, being shown the position of predetermined critical structures (such as boundary 720) can help the surgeon avoid damaging these structures, such as blood vessels, nerves, healthy tissues, etc. Being able to update the location of the wireless marker 104 at frequent intervals can allow the surgeon to proceed at a normal pace, without having to wait for the first tracking system 106 to update the location of the wireless marker 104, even if the wireless marker 104 moves as a result of part of the body of the patient 102 being disturbed by the surgery. The time intervals mentioned above may be suitable for allowing the surgeon to proceed at a normal pace. The ability to update the location of the wireless marker 104 at such frequent intervals can be an advantage of a tracking system, such as a radioactive tracking system, over a radioactive imaging system, which is sometimes used to image an organ or a tumor which has taken up radioactive material. Such radioactive imaging can yield imaging data at a slower rate than rate of tracking data associated with radioactive tracking. For example, it can take several minutes to acquire an image using radioactive imaging techniques.

At Step 626, when the wireless marker 104 becomes visible to the surgeon, either directly or through an endoscope, he or she can proceed with the surgery, for example excising or ablating a tumor or other tissue that was marked by the wireless marker 104, or taking a biopsy sample, and treating the marked tissue, for example with a locally administered drug. Optionally, the visual aid continues to provide images until the guided surgery system 100, 300, 400, and/or 500 is turned off.

It is expected that during the life of a patent maturing from this application many relevant medical imaging modalities, as well as many relevant tracking systems, will be developed and the scope of the terms imaging detector, imager, and tracking system are intended to include all such new technologies a priori.

As used herein the adjective "real-time" as applied to tracking systems means a tracking system configured to provide or generate tracking data on a tracked object, where the object is being manipulated or used by a user, at a rate sufficient for the provided or generated tracking data to be used as feed back to the user during the time that the user is engaged in manipulating or using the tracked object.

As used herein the noun "real time" in connection with a system or process where a user is provided with data processed by the system that is tracking an object manipulated or used by the user means the time period during which the user is manipulating or using the object.

As used herein the term "about" refers to ± 10 %.

As used herein, the term "endoscope" includes laparoscopes, thoracoscopes, and similar instruments that produce visual images from inside the body, including robotic instruments which are controlled by a surgeon.

The terms "comprises", "comprising", "includes", "including", "having" and their conjugates mean "including but not limited to". This term encompasses the terms "consisting of" and "consisting essentially of".

The phrase "consisting essentially of" means that the composition or method may include additional ingredients and/or steps, but only if the additional ingredients and/or steps do not materially alter the basic and novel characteristics of the claimed composition or method.

As used herein, the singular form "a", "an" and "the" include plural references unless the context clearly dictates otherwise. For example, the term "a compound" or "at least one compound" may include a plurality of compounds, including mixtures thereof.

The word "exemplary" is used herein to mean "serving as an example, instance or illustration". Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments". Any particular embodiment consistent with the disclosure may include a plurality of "optional" features unless such features conflict.

Throughout this application, various embodiments may be presented in a range format. It should be understood that the description in range format is merely for convenience and brevity and should not be construed as an inflexible limitation on the scope of the inventions. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features consistent with the disclosure, which are, for clarity, described in the context of separate embodiments, may also be provided in combination in a single embodiment. Conversely, various features consistent with the disclosure, which are, for brevity, described in the context of a single embodiment, may also be provided separately or in any suitable subcombination or as suitable in any other described embodiment consistent with the disclosure. Certain features described in the context of various embodiments are not to be considered essential features of those embodiments, unless the embodiment is inoperative without those elements.

Although specific embodiments consistent with the disclosure have been described, it is evident that many alternatives, modifications and variations will be apparent to those skilled in the art. Accordingly, it is intended to embrace all such alternatives, modifications and variations that fall within the spirit and broad scope of the appended claims.

Other embodiments of the invention will be apparent to those skilled in the art from consideration of the specification and practice of the invention disclosed herein. It is intended that the specification and examples be considered as exemplary only, with a true scope and spirit of the invention being indicated by the following claims.

## Claims

1. A surgical tracking system, comprising:
at least one sensor:
adapted to acquire a marker signal associated with a first location of a marker within a body and to output a first signal indicative of the first location, and
adapted to acquire a device signal associated with a second location of a movable imaging device and to output a second signal indicative of the second location, the movable imaging device being configured to generate a plurality of sequential images; and
at least one processor configured to receive data associated with the first signal, data associated with the second signal, and data associated with the plurality of sequential images, and to generate data associated with a plurality of composite images, where at least one of the plurality of composite images include a visual indication of the first location on at least one of the plurality of sequential images.

2. The surgical tracking system of claim 1, wherein the sequential composite images are configured to be generated multiple times per second.

3. The surgical tracking system of claim 2, wherein the sequential composite images are configured to be generated substantially in real time.

4. The surgical tracking system of claim 1, wherein the at least one sensor includes a first sensor adapted to acquire the marker signal associated with the first location of the marker, and a second sensor adapted to acquire the device signal associated with the second location of the movable imaging device.

5. The surgical tracking system of claim 1, wherein the marker emits ionizing radiation and wherein the at least one sensor is configured to detect ionizing radiation.

6. The surgical tracking system of claim 4, wherein the wherein the first sensor is adapted to detect ionizing radiation, and wherein the second sensor is adapted to detect non-ionizing radiation.

7. The surgical tracking system of claim 6, wherein the first sensor and the second sensor are each wireless sensors.

8. The surgical tracking system of claim 1, wherein the movable imaging device includes a camera.

9. The surgical tracking system of claim 8, wherein the camera is an intra-body camera.

10. The surgical tracking system of claim 1, wherein the first signal changes over time as the marker moves and wherein the second signal changes over time as the imaging device moves, and wherein the processor is configured to account for relative movement of the marker and the imaging device in generating the data associated with the plurality of composite images.

11. The surgical tracking system of claim 8, wherein the at least one sensor includes a first sensor is adapted to detect ionizing radiation and a second sensor is adapted to detect non-ionizing radiation.

12. A method of guided surgery, the method comprising:
implanting a marker inside a body;
tracking a first location of the marker;
acquiring an image of at least a portion of the body using an imaging device;
tracking a second location of the imaging device; and
displaying a composite image including the image of at least a portion of the body and an indication of a marker location;
wherein tracking the first location of the marker includes using a first sensor to acquire a marker signal associated with the first location of the marker, and
wherein tracking the second location of the imaging device includes using a second sensor to acquire a device signal associated with the second location of the imaging device.

13. The method according to claim 12, wherein the marker includes a source of ionizing radiation.

14. The method according to claim 12, wherein the first sensor is configured to detect a source of ionizing radiation.

15. The method according to claim 12, wherein the step of acquiring an image of at least a portion of the body using an imaging device and the step of displaying the composite image of the marker relative to the image of at least a portion of the body occur substantially in real time.

16. The method according to claim 12, wherein the imaging device is an intra-body imaging device.

17. The method according to claim 12, wherein the imaging device is an extra-body imaging device.

18. The method according to claim 12, wherein acquiring an image of at least a portion of the body includes capturing a plurality of sequential images.

19. The method according to claim 12, wherein displaying the composite image includes computing at least one coordinate transformation between a first set of coordinates associated with the first location of the marker and a second set of coordinates associated with the second location of the imaging device.

20. The method according to claim 19, wherein displaying the composite image further includes displaying an indication of a margin associated with a defined internal region of the body.

21. The method according to claim 20, further including acquiring an image of at least a portion of the body associated with the defined internal region and combining the indication of the margin with the image of at least a portion of the body associated with the defined internal region.

22. The method according to claim 12, wherein the surgery comprises breast surgery, and the marker is implanted in a breast tumor.

23. The method according to claim 12, wherein the surgery comprises pulmonary surgery, and the marker is implanted in a pulmonary tumor.

24. The method according to claim 12, wherein the imaging device is a laparoscope.

25. The method according to claim 12, wherein the imaging device is a stereoscopic laparoscope.

26. A surgical tracking system, comprising:
a first sensor adapted to acquire a marker signal associated with a first location of a marker within a body and to output a first signal indicative of the first location, and
a second sensor adapted to acquire a device signal associated with a second location of a surgical tool proximal to the body and to output a second signal indicative of the second location; and
at least one processor configured to receive data associated with the first signal and data associated with the second signal, to compute at least one coordinate transformation between a first set of coordinates associated with the first location of the marker and a second set of coordinates associated with the second location of the surgical tool, and to generate data associated with an indication of the location of the surgical tool with respect to the marker.

27. The surgical tracking system of claim 26, wherein the first signal changes over time as the marker moves and wherein the second signal changes over time as the surgical tool moves, and wherein the processor is configured to account for relative movement of the marker and the surgical tool in the indication of the location of the surgical tool with respect to the marker.

28. The surgical tracking system of claim 26, wherein the marker emits ionizing radiation and wherein the first sensor is configured to detect ionizing radiation.

29. The surgical tracking system of claim 28, wherein the second sensor is configured to detect a source of non-ionizing radiation.

30. The surgical tracking system of claim 29, wherein the surgical tool is adapted for use inside the body, and the second sensor is further adapted to acquire the device signal when the surgical tool is used inside the body.

31. The surgical tracking system of claim 29, further comprising a third sensor adapted to acquire a second device signal associated with a third location of the surgical tool proximal to the body and to output a third signal indicative of the third location;
wherein the surgical tool is adapted for use inside the body, the second sensor is further adapted to acquire the device signal from at least a portion of the surgical tool outside the body, and the third sensor is further adapted to acquire the second device signal from at least a second portion of the surgical tool inside the body.

32. A surgical tracking system, comprising:
at least one sensor:
adapted to acquire a marker signal associated with a first location of a marker within a body and to output a first signal indicative of the first location, and
adapted to acquire a device signal associated with a second location of a movable display apparatus and to output a second signal indicative of the second location, the movable display apparatus device being configured to generate a plurality of sequential data sets, each of the plurality of sequential data sets associated with a point of view of the movable display apparatus; and
at least one processor configured to receive data associated with the first signal, data associated with the second signal, and data associated with the plurality of sequential data sets, and to generate data associated with a plurality of projected indications, where at least one of the plurality of projected indications include a visual indication of the first location associated with at least one of the points of view of the movable display apparatus.

33. The surgical tracking system of claim 32, wherein the sequential data sets are configured to be generated multiple times per second.

34. The surgical tracking system of claim 33, wherein the plurality of projected indications are configured to be generated substantially in real time.

35. The surgical tracking system of claim 32, wherein the at least one sensor includes a first sensor adapted to acquire the marker signal associated with the first location of the marker, and a second sensor adapted to acquire the device signal associated with the second location of the movable display apparatus.

36. The surgical tracking system of claim 32, wherein the marker emits ionizing radiation and wherein the at least one sensor is configured to detect ionizing radiation.

37. The surgical tracking system of claim 36, wherein the wherein the first sensor is adapted to detect ionizing radiation, and wherein the second sensor is adapted to detect non-ionizing radiation.

38. The surgical tracking system of claim 37, wherein the first sensor and the second sensor are each wireless sensors.

39. The surgical tracking system of claim 32, wherein the movable display apparatus includes a projection device.

40. The surgical tracking system of claim 39, wherein the projection device is configured to project the visual indication onto the body.

41. The surgical tracking system of claim 32, wherein the movable display apparatus is configured to be worn by a user and the display apparatus further includes a display portion that is at least partially see-through and that is configured to provide the user a view of the visual indication.

42. The surgical tracking system of claim 41, wherein the movable display apparatus is selected from one of the set of: a heads-up display and see-through eyewear.

43. The surgical tracking system of claim 32, wherein the first signal changes over time as the marker moves and wherein the second signal changes over time as the display apparatus moves, and wherein the processor is configured to account for relative movement of the marker and the display apparatus in generating the data associated with the plurality of projected indications.

44. A computer-readable medium storing a program for causing a computer to execute a method of guided surgery, the method comprising:
implanting a marker inside a body;
tracking a first location of the marker;
acquiring an image of at least a portion of the body using an imaging device;
tracking a second location of the imaging device; and
displaying a composite image including the image of at least a portion of the body and an indication of a marker location;
wherein tracking the first location of the marker includes using a first sensor to acquire a marker signal associated with the first location of the marker, and
wherein tracking the second location of the imaging device includes using a second sensor to acquire a device signal associated with the second location of the imaging device.

45. The computer-readable medium of claim 44, wherein the marker includes a source of ionizing radiation.

46. The computer-readable medium of claim 44, wherein the first sensor is configured to detect a source of ionizing radiation.

47. The computer-readable medium of claim 44, wherein the step of acquiring an image of at least a portion of the body using an imaging device and the step of displaying the composite image of the marker relative to the image of at least a portion of the body occur substantially in real time.

48. The computer-readable medium of claim 44, wherein the imaging device is an intra-body imaging device.

49. The computer-readable medium of claim 44, wherein the imaging device is an extra-body imaging device.

50. The computer-readable medium of claim 44, wherein acquiring an image of at least a portion of the body includes capturing a plurality of sequential images.

51. The computer-readable medium of claim 44, wherein displaying the composite image includes computing at least one coordinate transformation between a first set of coordinates associated with the first location of the marker and a second set of coordinates associated with the second location of the imaging device.

52. The computer-readable medium of claim 51, wherein displaying the composite image further includes displaying an indication of a margin associated with a defined internal region of the body.

53. The computer-readable medium of claim 52, wherein the method further comprises: acquiring an image of at least a portion of the body associated with the defined internal region and combining the indication of the margin with the image of at least a portion of the body associated with the defined internal region.

54. The computer-readable medium of claim 44, wherein the surgery comprises breast surgery, and the marker is implanted in a breast tumor.

55. The computer-readable medium of claim 44, wherein the surgery comprises pulmonary surgery, and the marker is implanted in a pulmonary tumor.

56. The computer-readable medium of claim 44, wherein the imaging device is a laparoscope.

57. The computer-readable medium of claim 44, wherein the imaging device is a stereoscopic laparoscope.
